# EUROPÄISCHE PATENTSCHRIFT

(11) **EP 4 511 111 B1**
(45) Veröffentlichungstag und Bekanntmachung des Hinweises auf die Patenterteilung: **19.11.2025**
(21) Anmeldenummer: 23715553.6
(22) Anmeldetag: 03.04.2023
(51) Int. Cl.: A61N 5/10

(54) **VERFAHREN UND SYSTEM ZUM UNTERSTÜTZEN DER POSITIONIERUNG EINES PATIENTEN**
METHOD AND SYSTEM FOR SUPPORTING THE PROCESS OF POSITIONING A PATIENT
PROCÉDÉ ET SYSTÈME PRENANT EN CHARGE UN PROCESSUS DE POSITIONNEMENT DE PATIENT

(30) Priorität: 19.04.2022 DE 102022109402
(43) Veröffentlichungstag der Anmeldung: 26.02.2025
(73) Patentinhaber: LAP GmbH Laser Applikationen, 21337 Lüneburg (DE)
(72) Erfinder: KAYSER, Daniel, Hamburg 20255 (DE); SPECK, Thomas, 68259 Mannheim (DE)
(74) Vertreter: Hauck Patent- und Rechtsanwälte PartmbB
(86) Internationale Anmeldenummer: PCT/EP2023/058683
(87) Internationale Veröffentlichungsnummer: WO 2023/202870

(56) Entgegenhaltungen:
- US-A1- 2010 208 274
- US-A1- 2011 135 190

## Beschreibung

Die Erfindung betrifft ein Verfahren und ein System zum Unterstützen der Positionierung eines Patienten zur Bestrahlung mit einem Bestrahlungsgerät in einem Behandlungsraum. Mit solchen Bestrahlungsgeräten werden in der Strahlentherapie zum Beispiel Tumore bestrahlt. Als Bestrahlungsgeräte kommen unter anderem Linearbeschleuniger (Linac) zum Einsatz. Ein wichtiger Aspekt in der Strahlentherapie ist die präzise Patientenpositionierung, sodass die durch das Bestrahlungsgerät erzeugte Strahlendosis das zu behandelnde Körpergewebe möglichst genau trifft.

Bekannt ist die Patientenpositionierung mithilfe von Lasern und Markierungen auf der Patientenhaut. Ein solches System ist zum Beispiel aus DE 195 24 951 A1 bekannt. Lasersysteme zur Projektion von Laserebenen für die Patientenpositionierung, insbesondere Coronal-, Transversal- und Sagittalebenen, existieren zum Beispiel in Untersuchungsräumen mit bildgebenden Geräten, zum Beispiel CT-Scannern für die CT-Simulation, sowie an Bestrahlungsgeräten. Beispielsweise im Verlauf einer CT-Simulation und darauffolgender Bestrahlungsplanung wird das Isozentrum des zu behandelnden Tumors im Körper des Patienten mithilfe von Bildgebung bestimmt. Dieses Isozentrum des Tumors soll zu einem späteren Zeitpunkt in der Regel in einem getrennten Behandlungsraum möglichst genau in Übereinstimmung gebracht werden mit dem Isozentrum des Bestrahlungsgeräts. Um die Isozentrumsposition des Tumors von außen am Patientenkörper sichtbar zu machen, werden in dem Untersuchungsraum mit dem bildgebenden System mindestens drei orthogonal zueinanderstehende Laserebenen so ausgerichtet, dass ihr gemeinsamer Schnittpunkt im Isozentrum des Tumors liegt. Für diese Ausrichtung können die Laserebenen mit verfahrbaren Laserschienen im Raum verschoben werden und/oder der Patient auf einer Patientenliege im Raum bewegt werden. Die projizierten Laserebenen zeichnen sich dabei als Linien auf der Oberfläche des Patientenkörpers ab, wobei jeweils zwei Laserlinien einen Schnittpunkt auf der Körperoberfläche bilden. Auf diese Weise können zum Beispiel drei Schnittpunkte aus jeweils zwei der drei orthogonalen Laserebenen auf der Patientenkörperoberfläche angezeigt werden, zum Beispiel auf zwei gegenüberliegenden Seiten und auf der Oberseite des Patientenkörpers. Die Schnittpunkte der Laserlinien können mittels Stiften oder auf die Haut aufgebrachten Markierungen markiert werden. Bei einer nachfolgenden Bestrahlung in dem Behandlungsraum kann wiederum anhand von drei orthogonal zueinander ausgerichteten Laserebenen durch den Schnittpunkt der drei Linien im Raum das Isozentrum des Bestrahlungsgeräts auf der Oberfläche des Patientenkörpers angezeigt werden. Um dieses Isozentrum des Bestrahlungsgeräts zu dem Isozentrum des Tumors auszurichten, kann der Patient beispielsweise mittels einer verfahrbaren Patientenliege so im Raum verschoben bzw. gedreht werden, dass die auf der Oberfläche des Patientenkörpers durch jeweils zwei der orthogonalen Laserebenen gebildeten Schnittpunkte mit den zuvor aufgebrachten Markierungen übereinstimmen. Wenn alle Schnittpunkte auf der Körperoberfläche mit den zuvor aufgebrachten Markierungen in Deckung gebracht wurden, ist die Patientenpositionierung abgeschlossen und die Bestrahlung kann beginnen. Ein ähnliches System ist in US 2011/135190 beschrieben.

Diese Methode der Patientenpositionierung ist für den Anwender einfach durchführbar und intuitiv, da unmittelbar erkennbar ist, welche Patientenbewegung nötig ist, um die Laserlinien und die Markierungen auf der Patientenoberfläche in Deckung und damit den Patienten in die gewünschte Position zu bringen. Ein Nachteil dieser Methode ist, dass der Patient mindestens für die Dauer der Bestrahlungsbehandlung, meist über mehrere Wochen, die Markierungen am Körper tragen muss. Es besteht die Gefahr, dass Markierungen abgewaschen oder anderweitig unkenntlich werden und damit eine Positionierung des Patienten nicht mehr hinreichend genau möglich ist. Auch können Veränderungen des Patientenkörpers während der Bestrahlungsbehandlung, zum Beispiel eine Gewichtsabnahme oder eine Veränderung der Rigidität oder Form des Patientenkörpers, nicht immer hinreichend genau erkannt und berücksichtigt werden. Außerdem werden nur drei ausgewählte Punkte für die Positionierung des Patienten genutzt, die auch außerhalb der Ebene der Schnittpunkte möglichst genau sein soll.

Aus dem Stand der Technik sind weiterhin 3D-Oberflächenerfassungssysteme bekannt, die eine Positionierung des Patienten für die Strahlentherapie ohne Markierungen auf dem Körper erlauben. Oberflächenerfassungssysteme sind zum Beispiel beschrieben in US 7 348 974 B2, US 7 889 906 B2, US 9 028 422 B2, US 2015/265852 A1 oder US 2016/129283 A1. Bei Oberflächenerfassungssystemen wird eine live gemessene dreidimensionale Oberfläche des Patientenkörpers mit einer beispielsweise zuvor im Rahmen einer Bestrahlungsplanung, zum Beispiel anhand einer CT-Simulation, erstellten dreidimensionalen Referenzoberfläche des Patientenkörpers verglichen. Abweichungen werden in dreidimensionalen Visualisierungen von Soll- und Ist-Oberfläche angezeigt. Zudem können Werte für erforderliche Verschiebungen und Rotationen des Patienten berechnet werden, die auf die Patientenposition angewendet werden müssen, damit die Soll- und Ist-Oberfläche übereinstimmen. Die Zielgenauigkeit der Patientenpositionierung ist mit solchen dreidimensionalen Oberflächenerfassungssystemen häufig größer, weil nicht nur entlang von projizierten Linien oder Kreuzungspunkten, sondern anhand gesamter Oberflächen oder Teilbereichen der Oberfläche ausgerichtet werden kann. Allerdings ist die Interpretation der durch das System angezeigten Werte in Bezug auf die Positionierung des Patienten weniger intuitiv und erfordert eine größere Gewöhnung und Lernphase des Anwenders. Hierdurch kann es auch zu Bedienfehlern kommen.

Ausgehend von dem erläuterten Stand der Technik liegt der Erfindung daher die Aufgabe zugrunde, ein Verfahren und ein System der eingangs genannten Art bereitzustellen, mit denen in für den Anwender einfacher und intuitiver Weise bei gleichzeitig größtmöglicher Genauigkeit die Positionierung eines Patienten zur Bestrahlung an einem Bestrahlungsgerät unterstützt wird.

Die Erfindung löst die Aufgabe durch die unabhängigen Ansprüche 1 und 13. Vorteilhafte Ausgestaltungen finden sich in den abhängigen Ansprüchen, der Beschreibung und den Figuren.

Für ein Verfahren der eingangs genannten Art löst die Erfindung die Aufgabe durch die Schritte:
- mit einem 3D-Oberflächenerfassungssystem wird eine aktuelle Oberfläche des Patientenkörpers, vorzugsweise in dem Behandlungsraum erfasst,
- mindestens ein das Isozentrum des Bestrahlungsgeräts anzeigender Schnittpunkt von mindestens zwei Linien mit der erfassten Oberfläche des Patientenkörpers wird berechnet,
- anhand einer Referenzoberfläche des Patientenkörpers, welche die Sollposition des Patientenkörpers zu dem Isozentrum des Bestrahlungsgeräts angibt, wird eine erforderliche Transformation berechnet, um die erfasste Oberfläche des Patientenkörpers und die Referenzoberfläche in Übereinstimmung zu bringen,
- auf Grundlage der berechneten Transformation wird eine Zielposition des mindestens einen das Isozentrum des Bestrahlungsgeräts anzeigenden Schnittpunkts mit der erfassten Oberfläche des Patientenkörpers bestimmt,
- in einem Visualisierungssystem werden der mindestens eine das Isozentrum des Bestrahlungsgeräts anzeigende Schnittpunkt mit der erfassten Oberfläche des Patientenkörpers und seine Zielposition angezeigt.

Die Erfindung löst die Aufgabe außerdem durch ein System der eingangs genannten Art,
- umfassend ein 3D-Oberflächenerfassungssystem, ausgebildet zum Erfassen einer aktuellen Oberfläche des Patientenkörpers, vorzugsweise in dem Behandlungsraum,
- umfassend eine Auswerteeinrichtung, ausgebildet zum Berechnen mindestens eines das Isozentrum des Bestrahlungsgeräts anzeigenden Schnittpunkts von mindestens zwei Linien mit der erfassten Oberfläche des Patientenkörpers,
- wobei die Auswerteeinrichtung weiter ausgebildet ist, anhand einer Referenzoberfläche des Patientenkörpers, welche die Sollposition des Patientenkörpers zu dem Isozentrum des Bestrahlungsgeräts angibt, eine erforderliche Transformation zu berechnen, um die erfasste Oberfläche des Patientenkörpers und die Referenzoberfläche in Übereinstimmung zu bringen,
- wobei die Auswerteeinrichtung weiter ausgebildet ist, auf Grundlage der berechneten Transformation eine Zielposition des mindestens einen das Isozentrum des Bestrahlungsgeräts anzeigenden Schnittpunkts mit der erfassten Oberfläche des Patientenkörpers zu bestimmen,
- und umfassend ein Visualisierungssystem, das ausgebildet ist, auf Grundlage der berechneten Transformation den mindestens einen das Isozentrum des Bestrahlungsgeräts anzeigenden Schnittpunkt mit der erfassten Oberfläche des Patientenkörpers und seine Zielposition anzuzeigen.

Das Bestrahlungsgerät dient zur Bestrahlung eines Patienten, zum Beispiel zur Tumorbehandlung. Es kann sich um einen Linearbeschleuniger (Linac) handeln. Erfindungsgemäß wird mit einem dreidimensionalen Oberflächenerfassungssystem die Position des vorzugsweise in dem Behandlungsraum zum Beispiel auf einer verfahrbaren Patientenliege befindlichen Patienten erfasst. Die Erfassung der dreidimensionalen Oberfläche des Patientenkörpers erfolgt insbesondere mehrfach nacheinander, zum Beispiel in festen Zeitabständen oder kontinuierlich. Das dreidimensionale Oberflächenerfassungssystem kann in an sich bekannter Weise ausgebildet sein. Es kann zum Beispiel Kameras umfassen, insbesondere ein stereoskopisches Kamerasystem, wie an sich bekannt.

Im Isozentrum des Bestrahlungsgeräts ist die von dem Bestrahlungsgerät erzeugte Strahlendosis oftmals am größten. Das Isozentrum des Bestrahlungsgeräts soll möglichst genau mit dem Isozentrum des zu bestrahlenden Gewebes, insbesondere eines Tumors, übereinstimmen. Hierzu wird erfindungsgemäß ein Schnittpunkt mindestens zweier Linien bzw. Ebenen auf der Körperoberfläche des Patienten berechnet. Dieser Schnittpunkt zeigt das Isozentrum des Bestrahlungsgeräts an. Er ergibt sich rechnerisch aus den Schnittlinien von sich im Isozentrum des Bestrahlungsgeräts schneidender orthogonaler Ebenen mit der Körperoberfläche. Das Isozentrum als Schnittpunkt zum Beispiel dreier orthogonaler Ebenen liegt üblicherweise im Inneren des auf der Patientenliege positionierten Patientenkörpers. Die beispielsweise drei orthogonalen Ebenen zeichnen sich daher als drei Linien auf dem Patientenkörper ab, die sich jeweils paarweise auf der Körperoberfläche schneiden, zum Beispiel auf zwei gegenüberliegenden Seiten und der Oberseite des Körpers. Jeweils zwei der drei Linien zeigen mit ihrem Schnittpunkt mit der Körperoberfläche also das Isozentrum an. Diese Linien und ihre Schnittpunkte können erfindungsgemäß berechnet und visualisiert werden. Entsprechend können drei Schnittpunkte von jeweils zwei Linien mit der erfassten Oberfläche des Patientenkörpers berechnet werden, nämlich die Schnittpunkte der drei sich im Isozentrum des Bestrahlungsgeräts schneidenden orthogonalen Ebenen an drei Positionen auf der Oberfläche des Patientenkörpers. Natürlich könnten auch mehr als drei Schnittpunkte auf der Körperoberfläche berechnet und visualisiert werden, zum Beispiel vier Schnittpunkte, wenn zum Beispiel auch ein Schnittpunkt auf der Unterseite des Patientenkörpers berechnet wird. Sofern vorliegend von Linien und ihren Schnittpunkten gesprochen wird, umfasst dies entsprechend auch die (orthogonalen) Ebenen, die sich als Linien auf der Körperoberfläche abzeichnen.

Bei der Erfindung wird weiterhin eine dreidimensionale Referenzoberfläche des Patientenkörpers berücksichtigt, die die Sollposition des Patientenkörpers zu dem Isozentrum des Bestrahlungsgeräts angibt. Die Referenzoberfläche gibt die Sollposition des Patientenkörpers für die Bestrahlung vor. Die Referenzoberfläche kann zum Beispiel im Rahmen einer der Bestrahlung vorangehenden Bestrahlungsplanung erstellt werden. Dazu kann mit einem bildgebenden Verfahren, wie zum Beispiel einem CT- oder MRT-Verfahren, das zu bestrahlende Gewebe, insbesondere der Tumor, ermittelt werden. Mit einem 3D-Oberflächenerfassungssystem zum Beispiel in einem Untersuchungsraum kann die Oberfläche des Patientenkörpers erfasst werden und es kann entsprechend berechnet werden, wie diese Oberfläche für die nachfolgende Bestrahlung in Bezug auf das Isozentrum des Bestrahlungsgeräts ausgerichtet sein muss. Das 3D-Oberflächenerfassungssystem in dem Untersuchungsraum kann dabei grundsätzlich in gleicher Weise ausgebildet sein wie das 3D-Oberflächenerfassungssystem im Behandlungsraum.

Auf Grundlage der so oder anderweitig, beispielsweise aus Daten eines bildgebenden Verfahrens, erstellten Referenzoberfläche wird eine Transformation der durch das 3D-Oberflächenerfassungssystem insbesondere im Behandlungsraum erfassten aktuellen Oberfläche des Patientenkörpers berechnet, um diese mit der Referenzoberfläche in Übereinstimmung zu bringen, insbesondere derart, dass das Isozentrum des Bestrahlungsgeräts mit dem Zentrum des zu bestrahlenden Gewebes des Patientenkörpers übereinstimmt. Die Transformation kann zum Beispiel eine erforderliche Verschiebung in sämtlichen drei Raumrichtungen und/oder eine Drehung des Patientenkörpers und/oder der Referenzoberfläche um eine oder mehrere Drehachsen umfassen. Die Berechnung der Transformation kann zum Beispiel mit an sich bekannten iterativen Methoden, wie der Methode des iterative closest point, erfolgen.

Erfindungsgemäß wird weiterhin auf Grundlage der berechneten Transformation eine Zielposition des mindestens einen das Isozentrum des Bestrahlungsgeräts anzeigenden Schnittpunkts mit der Oberfläche des Patientenkörpers bestimmt. In einem Visualisierungssystem werden der mindestens eine das Isozentrum des Bestrahlungsgeräts anzeigende Schnittpunkt mit der erfassten Oberfläche des Patientenkörpers und seine auf Grundlage der Transformation bestimmte Zielposition angezeigt. Auf Grundlage der Transformation bzw. einer auf dieser Grundlage durchgeführten inversen Transformation wird für den zuvor berechneten Schnittpunkt eine Zielposition im Koordinatensystem des Patienten bestimmt, wie sie sich auf dem Patientenkörper abzeichnen würde, wenn der Patientenkörper wie vorgegeben zum Isozentrum des Bestrahlungsgeräts ausgerichtet wäre. Die Zielposition entspricht dabei im Koordinatensystem des Patienten mindestens einem Punkt auf der Referenzoberfläche des Patientenkörpers. Auf Grundlage der erfindungsgemäß berechneten Transformation bzw. inversen Transformation kann grundsätzlich für beliebige Punkte auf der Oberfläche des Patientenkörpers, also unter anderem dem das Isozentrum anzeigenden Schnittpunkt der Linien bzw. orthogonalen Ebenen, eine solche Zielposition bestimmt werden. Auf Grundlage des Anzeigens des Schnittpunkts und der Zielposition in dem Visualisierungssystem kann ein Benutzer in einfacher Weise den Patienten so positionieren, dass der Schnittpunkt mit seiner Zielposition in Übereinstimmung gelangt und der Patient wie vorgegeben zum Isozentrum des Bestrahlungsgeräts ausgerichtet ist. Das Visualisierungssystem ist softwarebasiert. Es kann zum Beispiel auf einem PC, Laptop, Notebook, Tablet oder dergleichen ausgeführt werden. Die Visualisierung erfolgt entsprechend auf einem Display eines entsprechenden Geräts.

Sofern vorliegend von der Oberfläche oder der Referenzoberfläche des Patientenkörpers gesprochen wird, umfasst dies grundsätzlich die Oberfläche bzw. Referenzoberfläche des gesamten Patientenkörpers oder nur eines Abschnitts des Patientenkörpers, beispielsweise eines für die Bestrahlung interessierenden Abschnitts des Patientenkörpers. Weiter ist anzumerken, dass das erfindungsgemäße Verfahren vorzugsweise in dem Behandlungsraum mit dem Bestrahlungsgerät durchgeführt wird, bzw. dass sich das erfindungsgemäße System vorzugsweise in dem Behandlungsraum befindet. Zwingend ist dies jedoch nicht. So könnte zusätzlich oder alternativ das Verfahren auch in einem anderen Raum durchgeführt werden bzw. sich das System in einem anderen Raum befinden, zum Beispiel in einem Untersuchungsraum. Beispielsweise könnte das erfindungsgemäße Verfahren vor der Positionierung des Patienten in einem Behandlungsraum bereits in einem Untersuchungsraum durchgeführt werden.

Der Erfindung liegt somit der Gedanke zugrunde, anhand einer aktuell erfassten dreidimensionalen Oberfläche des Patientenkörpers und einer anhand einer dreidimensionalen Referenzoberfläche berechneten Transformation des Patientenkörpers virtuelle Markierungen auf der erfassten Oberfläche des Patientenkörpers und virtuelle Positionierungslaser zu berechnen und für den Anwender darzustellen. Die Patientenpositionierung ist auf Grundlage der virtuellen Markierungen und virtuellen Positionierungslaser besonders intuitiv und einfach durchführbar. Gleichzeitig werden die Vorteile der hochpräzisen Oberflächenerfassung und der daraus bestimmten Transformation ausgenutzt. Damit wird bei einfacher und intuitiver Bedienung, wie bei klassischen Markierungen auf der Körperoberfläche und entsprechenden Positionierungslasern, die Genauigkeit von 3D-Oberflächenerfassungssystemen erreicht. Die Vorteile beider Systeme werden kombiniert ohne die entsprechenden Nachteile zu übernehmen. Durch die vorzugsweise kontinuierliche Berechnung und Anzeige des mindestens einen Schnittpunkts und seiner Zielposition auf der Körperoberfläche wird dem Anwender in einfacher Weise visualisiert, in welchen Freiheitsgraden und Richtungen der Patient bewegt werden muss, damit der mindestens eine Schnittpunkt mit seiner Zielposition in Übereinstimmung gelangt.

Das erfindungsgemäße Verfahren, insbesondere seine Schritte, kann bzw. können mehrfach nacheinander durchgeführt werden, beispielsweise in festen Zeitabständen oder vorzugsweise kontinuierlich. Auf diese Weise ist eine fortlaufende Live-Erfassung der Patientenposition und erforderliche Neupositionierung des Patienten für die Bestrahlung in dem Visualisierungssystem anzeigbar und durch den Anwender kontrollierbar. Er kann so in einfacher Weise sukzessive die korrekte Patientenposition anfahren und/oder überwachen.

Zur Erleichterung der Positionierung können der mindestens eine Schnittpunkt und seine Zielposition in dem Visualisierungssystem als Kreuze von Linien angezeigt werden.

Nach einer Ausgestaltung kann eine den Patientenkörper tragende Patientenliege und/oder der Patient auf der Patientenliege so im Behandlungsraum bewegt werden, dass der mindestens eine das Isozentrum des Bestrahlungsgeräts anzeigende Schnittpunkt mit der erfassten Oberfläche des Patientenkörpers mit seiner Zielposition übereinstimmt. Die Positionierung kann automatisch oder manuell gesteuert erfolgen. Der Patientenkörper kann sich hierzu auf einer verfahrbaren Patientenliege befinden, die zum Beispiel entlang dreier senkrecht zueinander stehender Richtungen verfahrbar und außerdem um eine oder mehrere Drehachsen drehbar ist, z.B. eine vertikale Drehachse und eine Drehachse entlang einer Längs- und/oder Querrichtung der Patientenliege. Damit kann der Benutzer die Patientenliege bzw. den Patienten so positionieren, dass die Markierungen des Isozentrums des Bestrahlungsgeräts in dem Visualisierungssystem mit der Zielposition der Markierungen übereinstimmen.

Nach einer weiteren Ausgestaltung kann vorgesehen sein, dass mindestens drei das Isozentrum des Bestrahlungsgeräts anzeigende Schnittpunkte von mindestens drei Linien mit der erfassten Oberfläche des Patientenkörpers berechnet und in dem Visualisierungssystem angezeigt werden, und dass in dem Visualisierungssystem mindestens drei auf Grundlage der berechneten Transformation bestimmte Zielpositionen der mindestens drei das Isozentrum des Bestrahlungsgeräts anzeigenden Schnittpunkte mit der erfassten Oberfläche des Patientenkörpers angezeigt werden. Wie bereits erläutert, kann das Isozentrum des Bestrahlungsgeräts durch den Schnittpunkt dreier orthogonaler Ebenen insbesondere im Behandlungsraum definiert sein. Da sich dieses Isozentrum in der Regel innerhalb des Patientenkörpers befindet, bilden jeweils zwei der orthogonalen Ebenen als auf der Körperoberfläche abgebildete Linien einen Schnittpunkt auf der Oberfläche des Patientenkörpers, wobei durch drei orthogonale Ebenen insgesamt drei Schnittpunkte gebildet werden, beispielsweise auf gegenüberliegenden Seiten des Patientenkörpers und auf der Oberseite des Patientenkörpers. Diese Schnittpunkte können gemäß der vorgenannten Ausgestaltung berechnet und in dem Visualisierungssystem angezeigt werden. Entsprechend werden auf Grundlage der erfindungsgemäß berechneten Transformation auch die Zielpositionen dieser Schnittpunkte in der oben erläuterten Weise berechnet und in dem Visualisierungssystem angezeigt. Die Positionierung des Patientenkörpers wird durch diese Ausgestaltung weiter verbessert, indem durch eine entsprechende Verfahrbewegung der Patientenliege und/oder Bewegung des Patientenkörpers auf der Patientenliege sämtliche Schnittpunkte mit ihren Zielpositionen in Überdeckung gebracht werden müssen.

Gemäß einer weiteren besonders praxisgemäßen Ausgestaltung kann die erfasste Oberfläche des Patientenkörpers ebenfalls in dem Visualisierungssystem dargestellt werden, wobei der mindestens eine das Isozentrum anzeigende Schnittpunkt mit der erfassten Oberfläche des Patientenkörpers und seine Zielposition auf der im Visualisierungssystem dargestellten Oberfläche des Patientenkörpers angezeigt werden. Gleiches gilt natürlich für gegebenenfalls weitere Schnittpunkte. Die Darstellung des Patientenkörpers gemeinsam mit den Schnittpunkten und den entsprechenden Zielpositionen auf seiner Oberfläche vereinfacht die Visualisierung und damit die Positionierung des Patienten für den Benutzer weiter.

Nach einer weiteren Ausgestaltung können auch die den mindestens einen das Isozentrum des Bestrahlungsgeräts anzeigenden Schnittpunkt definierenden mindestens zwei Linien in dem Visualisierungssystem angezeigt werden. Auch die Zielposition des mindestens einen das Isozentrum des Bestrahlungsgeräts anzeigenden Schnittpunkts kann in dem Visualisierungssystem durch den Schnittpunkt mindestens zweier Linien angezeigt werden. Insbesondere kann die Anzeige der Linien auf der ebenfalls in dem Visualisierungssystem dargestellten aktuell erfassten Patientenoberfläche erfolgen. Durch die Anzeige und damit Ausrichtung der Linien im Zuge der Patientenpositionierung wird die Positioniergenauigkeit weiter verbessert, indem längere Linien, die sich insbesondere über die gesamte Länge und/oder Breite des Patientenkörpers erstrecken können, in Übereinstimmung gebracht werden müssen. Es erfolgt somit im Wesentlichen eine vollständige virtuelle Simulation der oben im Stand der Technik beschriebenen Patientenpositionierung anhand von projizierten orthogonalen Laserebenen und ihren auf der Patientenoberfläche als Linien dargestellten Schnitten mit derselben.

Die Referenzoberfläche des Patientenkörpers kann nach einer weiteren Ausgestaltung im Rahmen einer der Positionierung des Patienten vorangehenden Bestrahlungsplanung erstellt werden. Das Erstellen der Referenzoberfläche kann insbesondere in einem von dem Behandlungsraum getrennten Untersuchungsraum erfolgen. Dieser kann in an sich bekannter Weise ein bildgebendes System umfassen, zum Beispiel ein CT- oder MRT-System. Die Erfassung des Patientenkörpers erfolgt zusammen mit dem zu bestrahlenden Gewebe, zum Beispiel einem Tumor. Auf dieser Grundlage kann in an sich bekannter Weise die Referenzoberfläche erstellt werden.

Gemäß einer weiteren Ausgestaltung können über die das Isozentrum des Bestrahlungsgeräts anzeigenden Schnittpunkte hinaus weitere Schnittpunkte mit der erfassten Oberfläche des Patientenkörpers berechnet werden. Die weiteren Schnittpunkte können dann gemeinsam mit ihren auf Grundlage wiederum der berechneten Transformation bestimmten Zielpositionen in dem Visualisierungssystem angezeigt werden. Durch die virtuelle Markierung und Ausrichtung anhand weiterer Schnittpunkte, zum Beispiel markanter Punkte der Körperoberfläche, wie zum Beispiel Hüftknochen, Schädel, Nase, Füße, Kniegelenke etc. kann die Positionierung des Patienten im Raum weiter verbessert werden. Ähnlich wie bei einer Gliederpuppe können zum Beispiel Punkte auf der Oberfläche markiert werden, die eine Bewegung des Patientenkörpers oder eine Verformung des Patientenkörpers sichtbar machen, zum Beispiel im Bauchbereich oder dergleichen. Insbesondere eine Veränderung der Körperform, zum Beispiel wenn sich die Rigidität des Patientenkörpers verändert, wird so erkennbar. Dies würde zum Beispiel bei einer ausschließlichen Markierung des Isozentrums nicht immer zuverlässig erkannt. Auch die weiteren Schnittpunkte können Schnittpunkte jeweils zweier Linien bzw. orthogonaler Ebenen sein. Wiederum können auch die die weiteren Schnittpunkte definierenden Linien in dem Visualisierungssystem auf der Körperoberfläche dargestellt werden. Da erfindungsgemäß die Oberfläche des Patientenkörpers erfasst und für diese Oberfläche eine Transformation berechnet wird, können grundsätzlich beliebige Punkte auf der Körperoberfläche markiert und angezeigt werden. In der Praxis wird hier ein geeigneter Kompromiss aus Anzahl der anzuzeigenden und damit durch Verfahren der Patientenliege und/oder Bewegung des Patientenkörpers auf der Patientenliege in Überdeckung zu bringenden Punkten einerseits und erforderlicher Positioniergenauigkeit andererseits gewählt.

Um die Positionierung des Patienten weiter zu verbessern, können die weiteren Schnittpunkte zum Beispiel Schnittpunkte mit Randbereichen der Patientenoberfläche sein, zum Beispiel an gegenüberliegenden Enden des Patientenkörpers. Indem weit auseinanderliegende Markierungen mit ihren jeweiligen Zielpositionen in Übereinstimmung gebracht werden müssen, ist eine besonders genaue Positionierung möglich.

Nach einer weiteren Ausgestaltung können in dem Visualisierungssystem der mindestens eine Schnittpunkt und seine Zielposition aus verschiedenen Blickwinkeln angezeigt werden. Dies erleichtert die Positionierung weiter. Eine Anzeige kann zum Beispiel von gegenüberliegenden Seiten des Patientenkörpers und von einer Oberseite des Patientenkörpers erfolgen. Dies gilt entsprechend für die dreidimensionale Oberfläche des Patientenkörpers, sofern diese ebenfalls im Visualisierungssystem angezeigt wird.

Die Zielposition des mindestens einen das Isozentrum des Bestrahlungsgeräts anzeigenden Schnittpunkts mit der erfassten Oberfläche des Patientenkörpers kann nach einer weiteren Ausgestaltung anhand von in dem Behandlungsraum und/oder einem Untersuchungsraum angeordneten Linien- oder Kreuzlasern überprüft werden. Bei dieser Ausgestaltung werden in dem Behandlungsraum bzw. dem Untersuchungsraum angeordnete physische Laser, die orthogonale Laserebenen im Raum projizieren, genutzt, um die Position der virtuellen Lasermarkierungen zu definieren. Hierzu kann die aktuelle Position der im Behandlungsraum bzw. Untersuchungsraum befindlichen physischen Laser erfasst werden und in die virtuellen Oberflächendaten des erfindungsgemäßen Systems übertragen werden.

Nach einer weiteren Ausgestaltung kann mindestens eine weitere Zielposition mindestens eines weiteren Schnittpunkts mit der Oberfläche des Patientenkörpers bestimmt werden, und in dem Visualisierungssystem kann der mindestens eine weitere Schnittpunkt mit der erfassten Oberfläche des Patientenkörpers und seine weitere Zielposition angezeigt werden. Auf diese Weise können weitere virtuelle Schnittpunkte mit der erfassten Oberfläche des Patientenkörpers bestimmt und als Zielposition übernommen werden.

Nach einer weiteren Ausgestaltung kann mit in dem Behandlungsraum und/oder einem Untersuchungsraum angeordneten Linien- oder Kreuzlasern eine Position eines Schnittpunkts auf der erfassten Oberfläche des Patientenkörpers angezeigt werden, und die angezeigte Position als Zielposition oder als weitere Zielposition bestimmt und in dem Visualisierungssystem angezeigt werden. Es können also weitere Schnittpunkte mit der erfassten Oberfläche des Patientenkörpers durch physische Linien- oder Kreuzlaser angezeigt und durch das erfindungsgemäße System ausgelesen werden. Diese weiteren Schnittpunkte können dann als (weitere) Zielpositionen übernommen werden.

Ein Ausführungsbeispiel der Erfindung wird nachfolgend anhand von Figuren näher erläutert. Es zeigen schematisch:
- Figur 1: ein erfindungsgemäßes System in einer perspektivischen Ansicht,
- Figur 2: eine Darstellung in einem Visualisierungssystem des erfindungsgemäßen Systems in einem ersten Betriebszustand,
- Figur 3: die Darstellung aus Figur 2 in einem zweiten Betriebszustand, und
- Figur 4: die Darstellung aus Figur 2 in einem dritten Betriebszustand.

Soweit nichts anderes angegeben ist, bezeichnen in den Figuren gleiche Bezugszeichen gleiche Gegenstände.

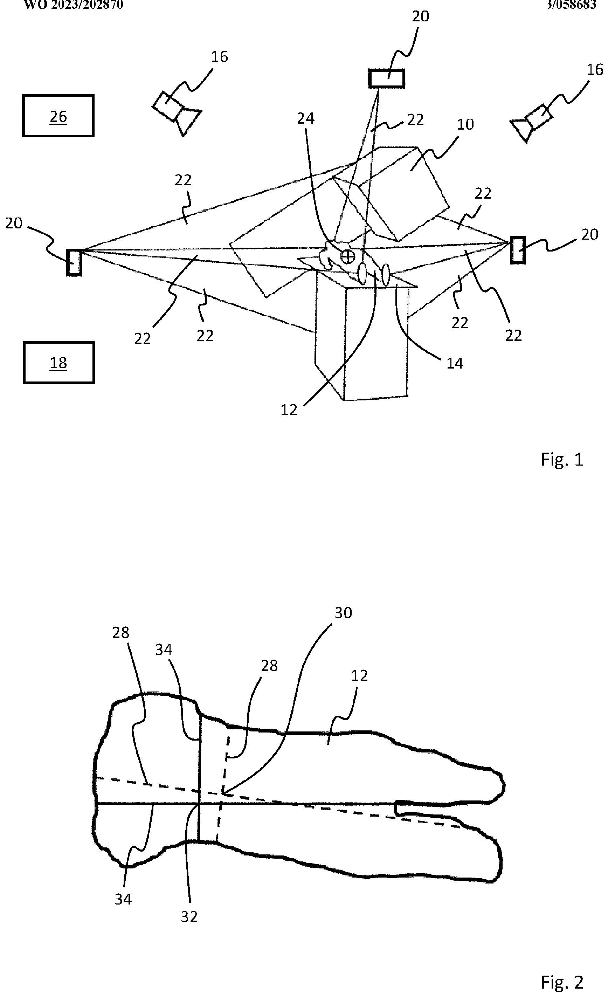

In Figur 1 ist das erfindungsgemäße System in einem Behandlungsraum mit einem Bestrahlungsgerät 10 dargestellt, bei dem es sich zum Beispiel um einen Linearbeschleuniger (Linac) zur Tumorbehandlung handeln kann. Ein Patientenkörper 12 befindet sich auf einer im Behandlungsraum verfahrbaren Patientenliege 14, die zum Beispiel entlang der drei Raumrichtungen verfahrbar ist und um eine vertikale Achse drehbar ist. Auch eine Drehbarkeit der Patientenliege 14 um ihre Längs- und/oder Querachse ist möglich. Das Bestrahlungsgerät 10 kann in an sich bekannter Weise während einer Bestrahlung um den Patientenkörper 12 rotiert werden.

Das System umfasst weiterhin ein 3D- Oberflächenerfassungssystem mit in dem dargestellten Beispiel zwei stereoskopisch arbeitenden Kameras 16. Mit den Kameras 16 wird die aktuelle dreidimensionale Oberfläche des Patientenkörpers 12 in dem Behandlungsraum erfasst. Dies kann mehrfach nacheinander, insbesondere kontinuierlich, erfolgen. Die erfasste Oberfläche des Patientenkörpers 12 wird in einem Visualisierungssystem 18 des erfindungsgemäßen Systems dargestellt, wie nachfolgend noch näher erläutert.

In dem Behandlungsraum sind weiterhin drei Linien- oder Kreuzlaser 20 angeordnet, die mindestens drei zueinander orthogonale Laserebenen 22 im Raum projizieren, die sich in dem Isozentrum 24 des Bestrahlungsgeräts 10 schneiden. Dieses Isozentrum 24 befindet sich regelmäßig im Inneren des Patientenkörpers 12. Die durch die Linien- oder Kreuzlaser 20 projizierten Laserebenen 22 bilden sich entsprechend linienförmig auf der Oberfläche des Patientenkörpers 12 ab, wobei jeweils zwei auf der Körperoberfläche abgebildete Laserlinien einen Schnittpunkt auf der Oberfläche des Patientenkörpers 12 bilden. Beispielsweise können so drei Schnittpunkte auf der Oberfläche des Patientenkörpers gebildet sein, nämlich auf gegenüberliegenden Seiten, in Figur 1 der linken und rechten Seite, des Patientenkörpers 12 sowie auf der Oberseite des Patientenkörpers 12.

Das erfindungsgemäße System umfasst weiterhin eine Auswerteeinrichtung 26, die unabhängig von den im Behandlungsraum physisch vorhandenen Linien- oder Kreuzlasern 20 die das Isozentrum 24 des Bestrahlungsgeräts 10 anzeigenden Schnittpunkte jeweils mindestens zweier Linien, entsprechend den von den physischen Linien- oder Kreuzlasern 20 projizierten Ebenen, mit der durch das 3D-Oberflächenerfassungssystem, insbesondere den Kameras 16, erfassten Oberfläche des Patientenkörpers 12 berechnet. Beispielsweise im Rahmen einer der Bestrahlung vorangehenden Bestrahlungsplanung mit einem bildgebenden System, wie einem CT- oder MRT-System, wird außerdem eine dreidimensionale Referenzoberfläche des Patientenkörpers erstellt, welche die Sollposition des Patientenkörpers 12 in Bezug auf das Isozentrum 24 des Bestrahlungsgeräts 10 angibt. Anhand dieser Referenzoberfläche wird durch die Auswerteeinrichtung 26 eine erforderliche Transformation berechnet, um die durch das 3D-Oberflächenerfassungssystem erfasste Oberfläche des Patientenkörpers 12 mit der Referenzoberfläche in Übereinstimmung zu bringen. Auf Grundlage der berechneten Transformation wird wiederum durch die Auswerteeinrichtung 26 eine Zielposition der das Isozentrum 24 des Bestrahlungsgeräts 10 anzeigenden Schnittpunkte mit der Oberfläche des Patientenkörpers 12 bestimmt. In der Regel ist die Sollposition des Patientenkörpers 12 dabei derart, dass das Isozentrum des zu behandelnden Gewebes, insbesondere des zu bestrahlenden Tumors, mit dem Isozentrum 24 des Bestrahlungsgeräts 10 zusammenfällt.

In dem Visualisierungssystem 18 werden die das Isozentrum 24 des Bestrahlungsgeräts 10 anzeigenden Schnittpunkte mit der erfassten Oberfläche des Patientenkörpers 12 und ihre jeweilige Zielposition angezeigt. Dies soll anhand der Figuren 2 bis 4 näher erläutert werden.

In Figur 2 ist ein Fall gezeigt, bei dem der Patientenkörper 12 noch nicht korrekt ausgerichtet ist. In dem dargestellten Beispiel werden in dem Visualisierungssystem 18 neben dem erfassten Patientenkörper 12 die Linien 28 angezeigt, die die das Isozentrum des Bestrahlungsgeräts anzeigenden Schnittpunkte definieren. In Figur 2 wird durch die Linien 28 der Schnittpunkt 30 als ein auf der Oberseite des Patientenkörpers 12 gebildeter Schnittpunkt definiert. Weitere Schnittpunkte sind in der Regel an gegenüberliegenden Seiten des Patientenkörpers gebildet. Wie in Figur 2 zu erkennen, wird in dem Visualisierungssystem 18 weiterhin die Zielposition 32 des Schnittpunkts 30 angezeigt, nämlich ebenfalls als Schnittpunkt 32 zweier Linien 34, also Ziellinien 34. In Figur 2 ist zu erkennen, dass der Patientenkörper noch nicht korrekt zur Bestrahlung positioniert ist. Der Schnittpunkt 30 und seine Zielposition 32 sowie die diese definierenden Linien 28, 34 befinden sich erkennbar noch nicht in Überdeckung.

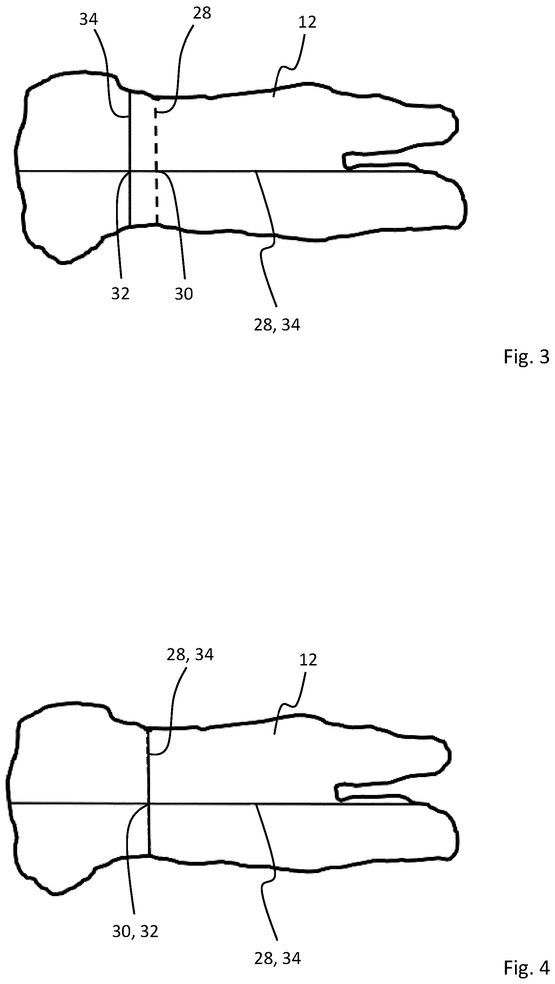

In Figur 3 ist ein erster Schritt der Patientenpositionierung erfolgt. Insbesondere ist eine Drehung des Patientenkörpers 12 und/oder der Patientenliege 14 um eine vertikale Drehachse erfolgt, sodass die in Figur 2 noch zu erkennende Verschiebung zwischen den Linien 28 und 34 behoben ist. Die in Längsrichtung verlaufenden Linien 28, 34 sind bereits zueinander ausgerichtet. Zwischen den quer zur Längsrichtung des Patientenkörpers 12 verlaufenden Linien 28, 34 besteht noch ein Abstand, der entsprechend auch zwischen dem Schnittpunkt 30 und seiner Zielposition 32 besteht.

In Figur 4 ist auch dieser Abstand behoben, indem der Patientenkörper 12 durch entsprechendes Verfahren der Patientenliege 14 und/oder Bewegen des Patientenkörpers auf der Patientenliege 14 in Längsrichtung so verschoben wurde, dass nunmehr sämtliche Linien 28, 34 und damit der Schnittpunkt 30 und seine Zielposition 32 in Überdeckung sind.

In entsprechender Weise erfolgt die Ausrichtung der weiteren Schnittpunkte der das Isozentrum 24 des Bestrahlungsgeräts 10 definierenden Linien für die vollständige Positionierung des Patientenkörpers 12. Die Patientenpositionierung ist abgeschlossen und die Bestrahlung kann erfolgen.

Auch ist es möglich, auf der Oberfläche des Patientenkörpers 12 weitere Schnittpunkte entsprechender Linien zu berechnen und darzustellen. Auf diese Weise kann die Positioniergenauigkeit weiter verbessert werden. In dem Visualisierungssystem 18 kann der Patientenkörper 12 mit den Schnittpunkten 30, 32 und den Linien 28, 34 entsprechend aus verschiedenen Blickwinkeln angezeigt werden, um eine Ausrichtung sämtlicher Schnittpunkt zu vereinfachen.

Sofern gewünscht, kann mittels der in dem Behandlungsraum angeordneten physischen Linien- oder Kreuzlaser 20 die Ausrichtung der berechneten Linien 28, 34, die in dem Visualisierungssystem 18 dargestellt werden, verifiziert werden.

### Bezugszeichenliste

- 10: Bestrahlungsgerät
- 12: Patientenkörper
- 14: Patientenliege
- 16: Kameras
- 18: Visualisierungssystem
- 20: Linien- oder Kreuzlaser
- 22: Laserebenen
- 24: Isozentrum des Bestrahlungsgeräts
- 26: Auswerteeinrichtung
- 28, 34: Linien
- 30: Schnittpunkt
- 32: Zielposition des Schnittpunkts

## Patentansprüche

1. Verfahren zum Unterstützen der Positionierung eines Patienten zur Bestrahlung mit einem Bestrahlungsgerät (10) in einem Behandlungsraum, umfassend die Schritte:
• mit einem 3D-Oberflächenerfassungssystem (16) wird eine aktuelle Oberfläche des Patientenkörpers (12) erfasst,
• mindestens ein das Isozentrum (24) des Bestrahlungsgeräts (10) anzeigender Schnittpunkt (30) von mindestens zwei Linien (28) mit der erfassten Oberfläche des Patientenkörpers (12) wird berechnet,
• anhand einer Referenzoberfläche des Patientenkörpers (12), welche die Sollposition des Patientenkörpers (12) zu dem Isozentrum (24) des Bestrahlungsgeräts (10) angibt, wird eine erforderliche Transformation berechnet, um die erfasste Oberfläche des Patientenkörpers (12) und die Referenzoberfläche in Übereinstimmung zu bringen,
• auf Grundlage der berechneten Transformation wird eine Zielposition (32) des mindestens einen das Isozentrum (24) des Bestrahlungsgeräts (10) anzeigenden Schnittpunkts (30) mit der erfassten Oberfläche des Patientenkörpers (12) bestimmt,
• in einem Visualisierungssystem (18) werden der mindestens eine das Isozentrum (24) des Bestrahlungsgeräts (10) anzeigende Schnittpunkt (30) mit der erfassten Oberfläche des Patientenkörpers (12) und seine Zielposition (32) angezeigt.

2. Verfahren nach Anspruch 1, **dadurch gekennzeichnet, dass** eine den Patientenkörper (12) tragende Patientenliege (14) und/oder der Patient auf der Patientenliege (14) so im Behandlungsraum bewegt wird, dass der mindestens eine das Isozentrum (24) des Bestrahlungsgeräts (10) anzeigende Schnittpunkt (30) mit der erfassten Oberfläche des Patientenkörpers (12) mit seiner Zielposition (32) übereinstimmt.

3. Verfahren nach einem der vorhergehenden Ansprüche, **dadurch gekennzeichnet, dass** mindestens drei das Isozentrum (24) des Bestrahlungsgeräts (10) anzeigende Schnittpunkte (30) von mindestens drei Linien (28) mit der erfassten Oberfläche des Patientenkörpers (12) berechnet und in dem Visualisierungssystem (18) angezeigt werden, und dass in dem Visualisierungssystem (18) mindestens drei auf Grundlage der berechneten Transformation bestimmte Zielpositionen (32) der mindestens drei das Isozentrum (24) des Bestrahlungsgeräts (10) anzeigenden Schnittpunkte (30) mit der erfassten Oberfläche des Patientenkörpers (12) angezeigt werden und/oder dass die erfasste Oberfläche des Patientenkörpers (12) ebenfalls in dem Visualisierungssystem (18) dargestellt wird, wobei der mindestens eine das Isozentrum (24) des Bestrahlungsgeräts (10) anzeigende Schnittpunkt (30) mit der erfassten Oberfläche des Patientenkörpers (12) und seine Zielposition (32) auf der im Visualisierungssystem (18) dargestellten Oberfläche des Patientenkörpers (12) angezeigt werden und/oder dass die den mindestens einen das Isozentrum (24) des Bestrahlungsgeräts (10) anzeigenden Schnittpunkt (30) definierenden mindestens zwei Linien (28) in dem Visualisierungssystem (18) angezeigt werden, und dass die Zielposition (32) des mindestens einen das Isozentrum (24) des Bestrahlungsgeräts (10) anzeigenden Schnittpunkts (30) ebenfalls durch mindestens zwei Linien (34) in dem Visualisierungssystem (18) angezeigt wird.

4. Verfahren nach einem der vorhergehenden Ansprüche, **dadurch gekennzeichnet, dass** die Referenzoberfläche des Patientenkörpers (12) im Rahmen einer der Positionierung des Patienten (12) in dem Behandlungsraum vorangehenden Bestrahlungsplanung erstellt wird.

5. Verfahren nach einem der vorhergehenden Ansprüche, **dadurch gekennzeichnet, dass** weitere Schnittpunkte (30) mit der erfassten Oberfläche des Patientenkörpers (12) berechnet werden, und dass die weiteren Schnittpunkte (30) sowie auf Grundlage der berechneten Transformation bestimmte Zielpositionen (32) der weiteren Schnittpunkte (30) in dem Visualisierungssystem (18) angezeigt werden, vorzugsweise wobei die weiteren Schnittpunkte (30) Schnittpunkte mit Randbereichen der Patientenoberfläche sind.

6. Verfahren nach einem der vorhergehenden Ansprüche, **dadurch gekennzeichnet, dass** in dem Visualisierungssystem (18) der mindestens eine Schnittpunkt (30) und seine Zielposition (32) aus verschiedenen Blickwinkeln angezeigt werden.

7. Verfahren nach einem der vorhergehenden Ansprüche, **dadurch gekennzeichnet, dass** die Zielposition (32) des mindestens einen das Isozentrum (24) des Bestrahlungsgeräts (10) anzeigenden Schnittpunkts (30) mit der erfassten Oberfläche des Patientenkörpers (12) anhand von in dem Behandlungsraum und/oder einem Untersuchungsraum angeordneten Linien- oder Kreuzlasern (20) überprüft wird.

8. Verfahren nach einem der vorhergehenden Ansprüche, **dadurch gekennzeichnet, dass** mindestens eine weitere Zielposition mindestens eines weiteren Schnittpunkts mit der Oberfläche des Patientenkörpers (12) bestimmt wird, und dass in dem Visualisierungssystem (18) der mindestens eine weitere Schnittpunkt mit der erfassten Oberfläche des Patientenkörpers (12) und seine weitere Zielposition angezeigt werden und/oder dass mit in dem Behandlungsraum und/oder einem Untersuchungsraum angeordneten Linien- oder Kreuzlasern (20) eine Position eines Schnittpunkts auf der erfassten Oberfläche des Patientenkörpers (12) angezeigt wird, und dass die angezeigte Position als Zielposition oder als weitere Zielposition bestimmt und in dem Visualisierungssystem (18) angezeigt wird.

9. System zum Unterstützen der Positionierung eines Patienten zur Bestrahlung mit einem Bestrahlungsgerät (10) in einem Behandlungsraum,
• umfassend ein 3D-Oberflächenerfassungssystem, ausgebildet zum Erfassen einer aktuellen Oberfläche des Patientenkörpers (12),
• umfassend eine Auswerteeinrichtung (26), ausgebildet zum Berechnen mindestens eines das Isozentrum (24) des Bestrahlungsgeräts (10) anzeigenden Schnittpunkts (30) von mindestens zwei Linien (28) mit der erfassten Oberfläche des Patientenkörpers (12),
• wobei die Auswerteeinrichtung (26) weiter ausgebildet ist, anhand einer Referenzoberfläche des Patientenkörpers (12), welche die Sollposition des Patientenkörpers (12) zu dem Isozentrum des Bestrahlungsgeräts (10) angibt, eine erforderliche Transformation zu berechnen, um die erfasste Oberfläche des Patientenkörpers (12) und die Referenzoberfläche in Übereinstimmung zu bringen,
• wobei die Auswerteeinrichtung (26) weiter ausgebildet ist, auf Grundlage der berechneten Transformation eine Zielposition (32) des mindestens einen das Isozentrum (24) des Bestrahlungsgeräts (10) anzeigenden Schnittpunkts (30) mit der erfassten Oberfläche des Patientenkörpers (12) zu bestimmen,
• und umfassend ein Visualisierungssystem (18), das ausgebildet ist, auf Grundlage der berechneten Transformation den mindestens einen das Isozentrum (24) des Bestrahlungsgeräts (10) anzeigenden Schnittpunkt (30) mit der erfassten Oberfläche des Patientenkörpers (12) und seine Zielposition (32) anzuzeigen.

10. System nach Anspruch 9, **dadurch gekennzeichnet, dass** es weiterhin eine den Patientenkörper (12) tragende Patientenliege (14) umfasst, die so im Behandlungsraum bewegbar ist, dass der mindestens eine das Isozentrum (24) des Bestrahlungsgeräts (10) anzeigende Schnittpunkt (30) mit der erfassten Oberfläche des Patientenkörpers (12) mit seiner Zielposition (32) in Übereinstimmung bringbar ist.

11. System nach einem der Ansprüche 9 oder 10, **dadurch gekennzeichnet, dass** die Auswerteeinrichtung (26) und das Visualisierungssystem (18) ausgebildet sind, mindestens drei das Isozentrum (24) des Bestrahlungsgeräts (10) anzeigende Schnittpunkte (30) von mindestens drei Linien (28) mit der erfassten Oberfläche des Patientenkörpers (12) zu berechnen und in dem Visualisierungssystem (18) anzuzeigen, und dass das Visualisierungssystem (18) weiter ausgebildet ist, mindestens drei auf Grundlage der berechneten Transformation bestimmte Zielpositionen (32) der mindestens drei das Isozentrum (24) des Bestrahlungsgeräts (10) anzeigenden Schnittpunkte (30) mit der erfassten Oberfläche des Patientenkörpers (12) anzuzeigen und/oder dass das Visualisierungssystem (18) weiter ausgebildet ist, die erfasste Oberfläche des Patientenkörpers (12) darzustellen, und den mindestens einen das Isozentrum (24) des Bestrahlungsgeräts (10) anzeigenden Schnittpunkt (30) mit der erfassten Oberfläche des Patientenkörpers (12) und seine Zielposition (32) auf der im Visualisierungssystem (18) dargestellten Oberfläche des Patientenkörpers (12) anzuzeigen und/oder dass das Visualisierungssystem (18) weiter ausgebildet ist, die den mindestens einen das Isozentrum (24) des Bestrahlungsgeräts (10) anzeigenden Schnittpunkt (30) definierenden mindestens zwei Linien (28) anzuzeigen, und die Zielposition (32) des mindestens einen das Isozentrum (24) des Bestrahlungsgeräts (10) anzeigenden Schnittpunkts (30, 32) ebenfalls durch mindestens zwei Linien (34) anzuzeigen.

12. System nach einem der Ansprüche 9 bis 11, **dadurch gekennzeichnet, dass** es weiterhin ein Bestrahlungsplanungssystem umfasst, das ausgebildet ist, die Referenzoberfläche des Patientenkörpers (12) im Rahmen einer der Positionierung des Patienten (12) in dem Behandlungsraum vorangehenden Bestrahlungsplanung zu erstellen.

13. System nach einem der Ansprüche 9 bis 12, **dadurch gekennzeichnet, dass** die Auswerteeinrichtung (26) weiter ausgebildet ist, weitere Schnittpunkte (30) mit der erfassten Oberfläche des Patientenkörpers (12) zu berechnen, und dass das Visualisierungssystem (18) weiter ausgebildet ist, die weiteren Schnittpunkte (30) und auf Grundlage der berechneten Transformation bestimmte Zielpositionen (32) der weiteren Schnittpunkte (30) anzuzeigen, vorzugsweise wobei die weiteren Schnittpunkte (30) Schnittpunkte mit Randbereichen der Patientenoberfläche sind.

14. System nach einem der Ansprüche 9 bis 13, **dadurch gekennzeichnet, dass** es weiterhin in dem Behandlungsraum angeordnete Linien- oder Kreuzlaser (20) umfasst, und dass die Auswerteeinrichtung (26) weiter ausgebildet ist, die Zielposition des mindestens einen das Isozentrum (24) des Bestrahlungsgeräts (10) anzeigenden Schnittpunkts (30) mit der erfassten Oberfläche des Patientenkörpers (12) anhand der in dem Behandlungsraum und/oder einem Untersuchungsraum angeordneten Linien- oder Kreuzlaser (20) zu überprüfen.

15. System nach einem der Ansprüche 9 bis 14, **dadurch gekennzeichnet, dass** dass das Visualisierungssystem (18) ausgebildet ist, den mindestens einen Schnittpunkt (30) und seine Zielposition (32) aus verschiedenen Blickwinkeln anzuzeigen und/oder dass die Auswerteeinrichtung (26) weiter ausgebildet ist, mindestens eine weitere Zielposition mindestens eines weiteren Schnittpunkts mit der Oberfläche des Patientenkörpers (12) zu bestimmen, und dass das Visualisierungssystem (18) weiter ausgebildet ist, den mindestens einen weiteren Schnittpunkt mit der erfassten Oberfläche des Patientenkörpers (12) und seine weitere Zielposition anzuzeigen und/oder dass es weiterhin in dem Behandlungsraum angeordnete Linien- oder Kreuzlaser (20) umfasst, die ausgebildet sind, eine Position eines Schnittpunkts auf der erfassten Oberfläche des Patientenkörpers (12) anzuzeigen, und dass die Auswerteeinrichtung (26) weiter ausgebildet ist, die angezeigte Position als Zielposition oder als weitere Zielposition zu bestimmen, und dass das Visualisierungssystem (18) weiter ausgebildet ist, die Zielposition oder die weitere Zielposition anzuzeigen.

## Claims

1. A method for supporting the positioning of a patient for irradiation with an irradiation device (10) in a treatment room, comprising the following steps:
• a current surface of the patient's body (12) is detected using a 3D surface detection system (16),
• at least one point of intersection (30) of at least two lines (28) with the detected surface of the patient's body (12) that is indicative of the isocenter (24) of the irradiation device (10) is calculated,
• a required transformation is calculated based on a reference surface of the patient's body (12) that is indicative of the intended position of the patient's body (12) with respect to the isocenter (24) of the irradiation device (10) in order to bring the detected surface of the patient's body (12) and the reference surface into alignment,
• a target position (32) of the at least one point of intersection (30) with the detected surface of the patient's body (12) that is indicative of the isocenter (24) of the irradiation device (10) is determined on the basis of the calculated transformation,
• the at least one point of intersection (30) with the detected surface of the patient's body (12) that is indicative of the isocenter (24) of the irradiation device (10), and the target position (32) of the point of intersection are displayed in a visualization system (18).

2. The method according to claim 1, **characterized in that** an examination table (14) supporting the patient's body (12) and/or the patient on the examination table (14) is moved in the treatment room in such a way that the at least one point of intersection (30) with the detected surface of the patient's body (12) that is indicative of the isocenter (24) of the irradiation device (10) matches the target position (32) of the point of intersection.

3. The method according to one of the preceding claims, **characterized in that** at least three points of intersection (30) of at least three lines (28) with the detected surface of the patient's body (12) that are indicative of the isocenter (24) of the irradiation device (10) are calculated and displayed in the visualization system (18), and **in that** at least three target positions (32) determined on the basis of the calculated transformation for the at least three points of intersection (30) with the detected surface of the patient's body (12) that are indicative of the isocenter (24) of the irradiation device (10) are displayed in the visualization system (18) and/or **in that** the detected surface of the patient's body (12) is also represented in the visualization system (18), wherein the at least one point of intersection (30) with the detected surface of the patient's body (12) that is indicative of the isocenter (24) of the irradiation device (10), and the target position (32) of the point of intersection are displayed on the surface of the patient's body (12) represented in the visualization system (18) and/or **in that** the at least two lines (28) defining the at least one point of intersection (30) that is indicative of the isocenter (24) of the irradiation device (10) are displayed in the visualization system (18), and **in that** the target position (32) of the at least one point of intersection (30) that is indicative of the isocenter (24) of the irradiation device (10) is also displayed by means of at least two lines (34) in the visualization system (18).

4. The method according to one of the preceding claims, **characterized in that** the reference surface of the patient's body (12) is created within the scope of irradiation planning prior to positioning of the patient (12) in the treatment room.

5. The method according to one of the preceding claims, **characterized in that** further points of intersection (30) with the detected surface of the patient's body (12) are calculated, and **in that** the further points of intersection (30) as well as target positions (32) of the further points of intersection (30) determined on the basis of the calculated transformation are displayed in the visualization system (18), preferably wherein the further points of intersection (30) are points of intersection with edge regions of the surface of the patient.

6. The method according to one of the preceding claims, **characterized in that** the at least one point of intersection (30) and the target position (32) thereof are displayed in the visualization system (18) from various viewing angles.

7. The method according to one of the preceding claims, **characterized in that** the target position (32) of the at least one point of intersection (30) with the detected surface of the patient's body (12) that is indicative of the isocenter (24) of the irradiation device (10) is checked using line or cross lasers (20) arranged in the treatment room and/or in an examination room.

8. The method according to one of the preceding claims, **characterized in that** at least one further target position of at least one further point of intersection with the surface of the patient's body (12) is determined, and **in that** the at least one further point of intersection with the detected surface of the patient's body (12) and the further target position thereof are displayed in the visualization system (18) and/or **in that** a position of a point of intersection on the detected surface of the patient's body (12) is displayed using line or cross lasers (20) arranged in the treatment room and/or in an examination room, and **in that** the displayed position is determined as a target position or as a further target position and is displayed in the visualization system (18).

9. A system for supporting the positioning of a patient for irradiation with an irradiation device (10) in a treatment room,
• comprising a 3D surface detection system which is designed to detect a current surface of the patient's body (12),
• comprising an evaluation apparatus (26) which is designed to calculate at least one point of intersection (30) of at least two lines (28) with the detected surface of the patient's body (12) that is indicative of the isocenter (24) of the irradiation device (10),
• wherein the evaluation apparatus (26) is further designed to calculate a required transformation based on a reference surface of the patient's body (12) that is indicative of the intended position of the patient's body (12) with respect to the isocenter of the irradiation device (10) in order to bring the detected surface of the patient's body (12) and the reference surface into alignment,
• wherein the evaluation apparatus (26) is further designed to determine a target position (32) of the at least one point of intersection (30) with the detected surface of the patient's body (12) that is indicative of the isocenter (24) of the irradiation device (10) on the basis of the calculated transformation,
• and comprising a visualization system (18) which is designed to display the at least one point of intersection (30) with the detected surface of the patient's body (12) that is indicative of the isocenter (24) of the irradiation device (10), and the target position (32) of the point of intersection on the basis of the calculated transformation.

10. The system according to claim 9, **characterized in that** it further comprises an examination table (14) that supports the patient's body (12) and that can be moved in the treatment room in such a way that the at least one point of intersection (30) with the detected surface of the patient's body (12) that is indicative of the isocenter (24) of the irradiation device (10) can be brought into alignment with the target position (32) of the point of intersection.

11. The system according to one of claims 9 or 10, **characterized in that** the evaluation apparatus (26) and the visualization system (18) are designed to calculate at least three points of intersection (30) of at least three lines (28) with the detected surface of the patient's body (12) that are indicative of the isocenter (24) of the irradiation device (10) and to display them in the visualization system (18), and **in that** the visualization system (18) is further designed to display at least three target positions (32) determined on the basis of the calculated transformation for the at least three points of intersection (30) with the detected surface of the patient's body (12) that are indicative of the isocenter (24) of the irradiation device (10) and/or **in that** the visualization system (18) is further designed to represent the detected surface of the patient's body (12), and to display the at least one point of intersection (30) with the detected surface of the patient's body (12) that is indicative of the isocenter (24) of the irradiation device (10), and the target position (32) of the point of intersection on the surface of the patient's body (12) represented in the visualization system (18) and/or **in that** the visualization system (18) is further designed to display the at least two lines (28) defining the at least one point of intersection (30) that is indicative of the isocenter (24) of the irradiation device (10), and to also display the target position (32) of the at least one point of intersection (30, 32) that is indicative of the isocenter (24) of the irradiation device (10) by means of at least two lines (34).

12. The system according to one of claims 9 to 11, **characterized in that** it further comprises an irradiation planning system that is designed to create the reference surface of the patient's body (12) within the scope of irradiation planning prior to positioning of the patient (12) in the treatment room.

13. The system according to one of claims 9 to 12, **characterized in that** the evaluation apparatus (26) is further designed to calculate further points of intersection (30) with the detected surface of the patient's body (12), and **in that** the visualization system (18) is further designed to display the further points of intersection (30) and target positions (32) of the further points of intersection (30) determined on the basis of the calculated transformation, preferably wherein the further points of intersection (30) are points of intersection with edge regions of the surface of the patient.

14. The system according to one of claims 9 to 13, **characterized in that** it further comprises line or cross lasers (20) arranged in the treatment room, and **in that** the evaluation apparatus (26) is further designed to check the target position of the at least one point of intersection (30) with the detected surface of the patient's body (12) that is indicative of the isocenter (24) of the irradiation device (10) using the line or cross lasers (20) arranged in the treatment room and/or in an examination room.

15. The system according to one of claims 9 to 14, **characterized in that** the visualization system (18) is designed to display the at least one point of intersection (30) and the target position (32) thereof from various viewing angles and/or **in that** the evaluation apparatus (26) is further designed to determine at least one further target position of at least one further point of intersection with the surface of the patient's body (12), and **in that** the visualization system (18) is further designed to display the at least one further point of intersection with the detected surface of the patient's body (12) and the further target position thereof and/or **in that** it further comprises line or cross lasers (20) arranged in the treatment room that are designed to display a position of a point of intersection on the detected surface of the patient's body (12), and **in that** the evaluation apparatus (26) is further designed to determine the displayed position as a target position or as a further target position, and **in that** the visualization system (18) is further designed to display the target position or the further target position.

## Revendications

1. Procédé prenant en charge le positionnement d'un patient pour l'irradiation avec un appareil d'irradiation (10) dans une salle de soins, comportant les étapes suivantes :
• une surface actuelle du corps du patient (12) est détectée à l'aide d'un système de détection de surface 3D (16),
• au moins un point d'intersection (30) d'au moins deux lignes (28) avec la surface détectée du corps du patient (12), indiquant l'isocentre (24) de l'appareil d'irradiation (10), est calculé,
• une transformation nécessaire est calculée à l'aide d'une surface de référence du corps du patient (12), laquelle indique la position souhaitée du corps du patient (12) par rapport à l'isocentre (24) de l'appareil d'irradiation (10), afin de faire coïncider la surface détectée du corps du patient (12) et la surface de référence,
• une position cible (32) de l'au moins un point d'intersection (30) avec la surface détectée du corps du patient (12) indiquant l'isocentre (24) de l'appareil d'irradiation (10) est déterminée sur la base de la transformation calculée,
• l'au moins un point d'intersection (30) avec la surface détectée du corps du patient (12) indiquant l'isocentre (24) de l'appareil d'irradiation (10) ainsi que sa position cible (32) sont indiqués dans un système de visualisation (18).

2. Procédé selon la revendication 1, **caractérisé en ce qu'**un support de patient (14) portant le corps du patient (12) et/ou le patient sur le support de patient (14) sont déplacés de telle façon dans la salle de soins, que l'au moins un point d'intersection (30) avec la surface détectée du corps du patient (12) indiquant l'isocentre (24) de l'appareil d'irradiation (10) coïncide avec sa position cible (32).

3. Procédé selon l'une des revendications précédentes, **caractérisé en ce qu'**au moins trois points d'intersection (30) d'au moins trois lignes (28) avec la surface détectée du corps du patient (12), indiquant l'isocentre (24) de l'appareil d'irradiation (10), sont calculés et indiqués dans le système de visualisation (18), et **en ce qu'**au moins trois positions cible (32), déterminées sur la base de la transformation calculée, des au moins trois points d'intersection (30) avec la surface détectée du corps du patient (12) indiquant l'isocentre (24) de l'appareil d'irradiation (10) sont indiquées dans le système de visualisation (18), et/ou **en ce que** la surface détectée du corps du patient (12) est également représentée dans le système de visualisation (18), dans lequel l'au moins un point d'intersection (30) avec la surface détectée du corps du patient (12) indiquant l'isocentre (24) de l'appareil d'irradiation (10) ainsi que sa position cible (32) sont indiqués sur la surface du corps du patient (12) représentée dans le système de visualisation (18) et/ou **en ce que** les au moins deux lignes (28) définissant l'au moins un point d'intersection (30) indiquant l'isocentre (24) de l'appareil d'irradiation (10) sont indiquées dans le système de visualisation (18), et **en ce que** la position cible (32) de l'au moins un point d'intersection (30) indiquant l'isocentre (24) de l'appareil d'irradiation (10) est également indiquée par au moins deux lignes (34) dans le système de visualisation (18).

4. Procédé selon l'une des revendications précédentes, **caractérisé en ce que** la surface de référence du corps du patient (12) est établie dans le cadre d'une planification d'irradiation antérieure au positionnement du patient (12) dans la salle de soins.

5. Procédé selon l'une des revendications précédentes, **caractérisé en ce que** d'autres points d'intersection (30) avec la surface détectée du corps du patient (12) sont calculés, et **en ce que** les autres points d'intersection (30) ainsi que des positions cibles (32) des autres points d'intersection (30), déterminées sur la base de la transformation calculée, sont indiqués dans le système de visualisation (18), dans lequel les autres points d'intersection (30) sont de préférence des points d'intersection avec des régions de bord de la surface de patient.

6. Procédé selon l'une des revendications précédentes, **caractérisé en ce que** l'au moins un point d'intersection (30) et sa position cible (32) sont indiqués sous différents angles dans le système de visualisation (18).

7. Procédé selon l'une des revendications précédentes, **caractérisé en ce que** la position cible (32) de l'au moins un point d'intersection (30) avec la surface détectée du corps du patient (12) indiquant l'isocentre (24) de l'appareil d'irradiation (10) est vérifiée à l'aide de lasers à lignes ou en croix (20) agencés dans la salle de soins et/ou dans une salle d'examen.

8. Procédé selon l'une des revendications précédentes, **caractérisé en ce qu'**au moins une autre position cible d'au moins un autre point d'intersection avec la surface du corps du patient (12) est déterminée, et **en ce que** l'au moins un autre point d'intersection avec la surface détectée du corps du patient (12) et son autre position cible sont indiqués dans le système de visualisation (18) et/ou **en ce qu'**une position d'un point d'intersection est indiquée sur la surface détectée du corps du patient (12) avec des lasers à lignes ou en croix (20) agencés dans la salle de soins et/ou dans une salle d'examen, et **en ce que** la position indiquée est déterminée comme position cible ou comme autre position cible et indiquée dans le système de visualisation (18).

9. Système prenant en charge le positionnement d'un patient pour l'irradiation avec un appareil d'irradiation (10) dans une salle de soins,
• comportant un système de détection de surface 3D, conçu pour détecter une surface actuelle du corps du patient (12),
• comportant un dispositif d'évaluation (26) conçu pour calculer au moins un point d'intersection (30) d'au moins deux lignes (28) avec la surface détectée du corps du patient (12), indiquant l'isocentre (24) de l'appareil d'irradiation (10),
• dans lequel le dispositif d'évaluation (26) est en outre conçu pour calculer une transformation nécessaire, à l'aide d'une surface de référence du corps du patient (12), laquelle indique la position souhaitée du corps du patient (12) par rapport à l'isocentre de l'appareil d'irradiation (10), afin de faire coïncider la surface détectée du corps du patient (12) et la surface de référence,
• dans lequel le dispositif d'évaluation (26) est en outre conçu pour déterminer, sur la base de la transformation calculée, une position cible (32) de l'au moins un point d'intersection (30) avec la surface détectée du corps du patient (12) indiquant l'isocentre (24) de l'appareil d'irradiation (10),
• et comportant un système de visualisation (18), lequel est conçu pour indiquer l'au moins un point d'intersection (30) avec la surface détectée du corps du patient (12) indiquant l'isocentre (24) de l'appareil d'irradiation (10), ainsi que sa position cible (32).

10. Système selon la revendication 9, **caractérisé en ce que** celui-ci comporte en outre un support de patient (14) portant le corps du patient (12), lequel peut être déplacé de telle façon dans la salle de soins, que l'au moins un point d'intersection (30) avec la surface détectée du corps du patient (12), indiquant l'isocentre (24) de l'appareil d'irradiation (10), peut être mis en coïncidence avec sa position cible (32).

11. Système selon l'une des revendications 9 ou 10, **caractérisé en ce que** le dispositif d'évaluation (26) et le système de visualisation (18) sont conçus pour calculer au moins trois points d'intersection (30) d'au moins trois lignes (28) avec la surface détectée du corps du patient (12), indiquant l'isocentre (24) de l'appareil d'irradiation (10) et pour les indiquer dans le système de visualisation (18), et **en ce que** le système de visualisation (18) est en outre conçu pour indiquer au moins trois positions cible (32), déterminées sur la base de la transformation calculée, des au moins trois points d'intersection (30) avec la surface détectée du corps du patient (12) indiquant l'isocentre (24) de l'appareil d'irradiation (10) et/ou **en ce que** le système de visualisation (18) est en outre conçu pour représenter la surface détectée du corps du patient (12), et pour indiquer l'au moins un point d'intersection (30) avec la surface détectée du corps du patient (12) indiquant l'isocentre (24) de l'appareil d'irradiation (10) ainsi que sa position cible (32) sur la surface du corps du patient (12) représentée dans le système de visualisation (18) et/ou **en ce que** le système de visualisation (18) est en outre conçu pour indiquer les au moins deux lignes (28) définissant l'au moins un point d'intersection (30) indiquant l'isocentre (24) de l'appareil d'irradiation (10), et pour indiquer, également par au moins deux lignes (34), la position cible (32) de l'au moins un point d'intersection (30, 32) indiquant l'isocentre (24) de l'appareil d'irradiation (10).

12. Système selon l'une des revendications 9 à 11, **caractérisé en ce que** celui-ci comporte en outre un système de planification d'irradiation, lequel est conçu pour établir la surface de référence du corps du patient (12) dans le cadre d'une planification d'irradiation antérieure au positionnement du patient (12) dans la salle de soins.

13. Système selon l'une des revendications 9 à 12, **caractérisé en ce que** le dispositif d'évaluation (26) est en outre conçu pour calculer d'autres points d'intersection (30) avec la surface détectée du corps du patient (12), et **en ce que** le système de visualisation (18) est en outre conçu pour indiquer les autres points d'intersection (30) ainsi que des positions cibles (32) des autres points d'intersection (30), déterminées sur la base de la transformation calculée, dans lequel les autres points d'intersection (30) sont de préférence des points d'intersection avec des régions de bord de la surface de patient.

14. Système selon l'une des revendications 9 à 13, **caractérisé en ce que** celui-ci comporte en outre des lasers à lignes ou en croix (20) agencés dans la salle de soins, et **en ce que** le dispositif d'évaluation (26) est en outre conçu pour vérifier la position cible de l'au moins un point d'intersection (30) avec la surface détectée du corps du patient (12) indiquant l'isocentre (24) de l'appareil d'irradiation (10) à l'aide des lasers à lignes ou en croix (20) agencés dans la salle de soins et/ou dans une salle d'examen.

15. Système selon l'une des revendications 9 à 14, **caractérisé en ce que** le système de visualisation (18) est conçu pour indiquer l'au moins un point d'intersection (30) et sa position cible (32) sous différents angles et/ou **en ce que** le dispositif d'évaluation (26) est en outre conçu pour déterminer au moins une autre position cible d'au moins un autre point d'intersection avec la surface du corps du patient (12), et **en ce que** le système de visualisation (18) est en outre conçu pour indiquer l'au moins un autre point d'intersection avec la surface détectée du corps du patient (12) et son autre position cible et/ou **en ce que** celui-ci comporte en outre des lasers à lignes ou en croix (20) agencés dans la salle de soins, lesquels sont conçus pour indiquer une position d'un point d'intersection sur la surface détectée du corps du patient (12), et **en ce que** le dispositif d'évaluation (26) est en outre conçu pour déterminer la position indiquée comme position cible ou comme autre position cible, et **en ce que** le système de visualisation (18) est en outre conçu pour indiquer la position cible ou l'autre position cible.
